# EUROPÄISCHE PATENTSCHRIFT

(11) **EP 2 896 691 B1**
(45) Veröffentlichungstag und Bekanntmachung des Hinweises auf die Patenterteilung: **12.07.2017**
(21) Anmeldenummer: 15000331.7
(22) Anmeldetag: 18.09.2009
(51) Int. Cl.: C12N 9/18, C12N 9/10, C12P 7/06, C12N 15/52

(54) **Zusatzstoff für den enzymatischem Abbau von Mykotoxinen sowie Verwendung desselben**
Additive for the enzymatic decomposition of mycotoxins and use of the same
Additif pour la decomposition enzymatique de mycotoxines et son utilisation

(30) Priorität: 18.09.2008 AT 50108 U
(43) Veröffentlichungstag der Anmeldung: 22.07.2015
(62) Teilanmeldung aus: 09775638.1
(73) Patentinhaber: Erber Aktiengesellschaft, 3131 Getzersdorf bei Traismauer (AT)
(72) Erfinder: Moll, Wulf-Dieter, 2000 Stockerau (AT); Hartinger, Doris, 1200 Wien (AT); Grießler, Karin, 3140 Pottenbrunn (AT); Binder, Eva Maria, 3430 Tulin (AT); Schatzmayr, Gerd, 3430 Tulin (AT)
(74) Vertreter: Cunow, Gerda

(56) Entgegenhaltungen:
- WO-A1-99/02703
- WO-A2-00/04158
- WO-A2-2006/053357

### SEQUENCE LISTING

<110> Erber Aktiengesellschaft
<120> Verfahren zur Herstellung eines Zusatzstoffes für den enzymatischen Abbau von Mykotoxinen sowie Zusatzstoff und Verwendung desselben
<130> P05371EP
<140> A8024/2009
   <141> 2009-09-18
<160> 5
<170> PatentIn version 3.5
<210> 1
   <211> 15420
   <212> DNA
   <213> Sphingopyxis sp.
<220>
   <221> Seq ID 1 (fum)
   <222> (1)..(15420)
<300>
   <308> FJ426269
   <309> 2009-06-11
<313> (1)..(15420)
<400> 1
<210> 2
   <211> 1623
   <212> DNA
   <213> Sphingopyxis sp.
<220>
   <221> Seq ID 8 (fumD)
   <222> (1)..(1623)
<300>
   <308> FJ426269
   <309> 2009-06-11
<313> (1)..(1623)
<400> 2
   gtgaaagagc accaatgccg tggcggccgg gcgtcccccg ctgcgcccgc cacgtggctt      60
   gcgcggatca gcgtttcccg gggggcctcc gccatcgcct ggaccttcat gcttggcgca     120
   actgccattc ccgtggctgc gcaaactgac gatccgaagc tcgttcgtca tacccagtcg     180
   ggcgccgtcg agggcgtcga gggcgacgtc gagacttttt tgggaatacc cttcgcggct     240
   ccgccggtcg gcgacctgcg atggcggccg ccggctccgc cgagggcgtg ggcgggcacc     300
   agggacggcc gccgctttgc gcccgattgc atcgggaacg agcggcttag agaggggagc     360
   cgggctgccg ggacgagcga agactgcctc tatctgaata tctggtctcc caaacaggtc     420
   ggtaaggggg ggctccccgt catgatctgg gtttacggcg gtgggtttag cggcggttct     480
   ggcgcggtgc catattatga cggctctgcg ctcgcgcaga agggcgtggt ggtcgtcacg     540
   ttcaactatc gcgccgggat tctgggcttt cttgcccatc cggcgctttc aaaggaaagt     600
   ccgaatggcg tgtcgggcaa ctatggtctt ctcgacatgc tcgcggcgtt caaatgggtt     660
   cagaacaaca taagggagtt cggcggagac ccgaaccgtg tcacggtctt tggcgagtcc     720
   gccggcgcga gcgcgctcgg actgctcctg acctcgccgc tcagtgagag cgccttcaat     780
   caggcgatac tgcaaagtcc gggtctggcc aggccgctcg ccacgctttc tgaaagcgaa     840
   gcgaatgggc tggagctggg agccgatatt tctgctctac ggcgtgccga tgcgggcgaa     900
   ttgacgaaga tcgcgcaatc gcgaataccc atgtcgcgcc agttcaccaa gccgcggccg     960
   atgggtccga ttctggacgg ctatgttttg cgcacccttg acgtcgatgc cttcgccaag    1020
   ggggccttcc gcaagatacc cgttctggtc ggcggaaacg ccgacgaagg gcgcgctttt    1080
   acggatcgcc tgccggtcaa aacggtcctt gaatatcgag cctatctcac agaacaattt    1140
   ggtgacgagg cggacgcatg ggagcgttgt tatcccgcga actccgacgc cgacgtcccc    1200
   gccgccgttg cccgtctttt tggggatagt cagttcaaca acgggatcga gctgctctcg    1260
   gcagccttcg cgaaatggcg aacgccgctt tggagatatc gctttacggg cattccagga    1320
   gccggccgtc gccccgccac gcatggagac gaaattccct atgtcttcgc aaatctgggg    1380
   ccgtcgtccg tatctatgtt tgggtcgctc gaaggcggcg ccggggcgtc ggacatcaaa    1440
   cttgcgaccg aaatgtccgc ggcctgggtg agcttcgcgg tgcacggggt ccccgatcag    1500
   ggcacgaaat cgcactggcc gcgcttcgag cggcgagggg agatcatgac ttttggttcg    1560
   caggttggct ctggggaagg tcttggagtt tcgccgagca aagcctgcca accctcaaaa    1620
   tag                                                                  1623
<210> 3
   <211> 540
   <212> PRT
   <213> Sphingopyxis sp.
<220>
   <221> Seq ID 9 (FumD)
   <222> (1)..(540)
<300>
   <308> FJ426269
   <309> 2009-06-11
<313> (1)..(540)
<400> 3
   Val Lys Glu His Gln Cys Arg Gly Gly Arg Ala Ser Pro Ala Ala Pro
   1               5                   10                  15
   Ala Thr Trp Leu Ala Arg Ile Ser Val Ser Arg Gly Ala Ser Ala Ile
               20                  25                  30
   Ala Trp Thr Phe Met Leu Gly Ala Thr Ala Ile Pro Val Ala Ala Gln
           35                  40                  45
   Thr Asp Asp Pro Lys Leu Val Arg His Thr Gln Ser Gly Ala Val Glu
       50                  55                  60
   Gly Val Glu Gly Asp Val Glu Thr Phe Leu Gly Ile Pro Phe Ala Ala
   65                  70                  75                  80
   Pro Pro Val Gly Asp Leu Arg Trp Arg Pro Pro Ala Pro Pro Arg Ala
                   85                  90                  95
   Trp Ala Gly Thr Arg Asp Gly Arg Arg Phe Ala Pro Asp Cys Ile Gly
               100                 105                 110
   Asn Glu Arg Leu Arg Glu Gly Ser Arg Ala Ala Gly Thr Ser Glu Asp
           115                 120                 125
   Cys Leu Tyr Leu Asn Ile Trp Ser Pro Lys Gln Val Gly Lys Gly Gly
       130                 135                 140
   Leu Pro Val Met Ile Trp Val Tyr Gly Gly Gly Phe Ser Gly Gly Ser
   145                 150                 155                 160
   Gly Ala Val Pro Tyr Tyr Asp Gly Ser Ala Leu Ala Gln Lys Gly Val
                   165                 170                 175
   Val Val Val Thr Phe Asn Tyr Arg Ala Gly Ile Leu Gly Phe Leu Ala
               180                 185                 190
   His Pro Ala Leu Ser Lys Glu Ser Pro Asn Gly Val Ser Gly Asn Tyr
           195                 200                 205
   Gly Leu Leu Asp Met Leu Ala Ala Phe Lys Trp Val Gln Asn Asn Ile
       210                 215                 220
   Arg Glu Phe Gly Gly Asp Pro Asn Arg Val Thr Val Phe Gly Glu Ser
   225                 230                 235                 240
   Ala Gly Ala Ser Ala Leu Gly Leu Leu Leu Thr Ser Pro Leu Ser Glu
                   245                 250                 255
   Ser Ala Phe Asn Gln Ala Ile Leu Gln Ser Pro Gly Leu Ala Arg Pro
               260                 265                 270
   Leu Ala Thr Leu Ser Glu Ser Glu Ala Asn Gly Leu Glu Leu Gly Ala
           275                 280                 285
   Asp Ile Ser Ala Leu Arg Arg Ala Asp Ala Gly Glu Leu Thr Lys Ile
       290                 295                 300
   Ala Gln Ser Arg Ile Pro Met Ser Arg Gln Phe Thr Lys Pro Arg Pro
   305                 310                 315                 320
   Met Gly Pro Ile Leu Asp Gly Tyr Val Leu Arg Thr Leu Asp Val Asp
                   325                 330                 335
   Ala Phe Ala Lys Gly Ala Phe Arg Lys Ile Pro Val Leu Val Gly Gly
               340                 345                 350
   Asn Ala Asp Glu Gly Arg Ala Phe Thr Asp Arg Leu Pro Val Lys Thr
           355                 360                 365
   Val Leu Glu Tyr Arg Ala Tyr Leu Thr Glu Gln Phe Gly Asp Glu Ala
       370                 375                 380
   Asp Ala Trp Glu Arg Cys Tyr Pro Ala Asn Ser Asp Ala Asp Val Pro
   385                 390                 395                 400
   Ala Ala Val Ala Arg Leu Phe Gly Asp Ser Gln Phe Asn Asn Gly Ile
                   405                 410                 415
   Glu Leu Leu Ser Ala Ala Phe Ala Lys Trp Arg Thr Pro Leu Trp Arg
               420                 425                 430
   Tyr Arg Phe Thr Gly Ile Pro Gly Ala Gly Arg Arg Pro Ala Thr His
           435                 440                 445
   Gly Asp Glu Ile Pro Tyr Val Phe Ala Asn Leu Gly Pro Ser Ser Val
       450                 455                 460
   Ser Met Phe Gly Ser Leu Glu Gly Gly Ala Gly Ala Ser Asp Ile Lys
   465                 470                 475                 480
   Leu Ala Thr Glu Met Ser Ala Ala Trp Val Ser Phe Ala Val His Gly
                   485                 490                 495
   Val Pro Asp Gln Gly Thr Lys Ser His Trp Pro Arg Phe Glu Arg Arg
               500                 505                 510
   Gly Glu Ile Met Thr Phe Gly Ser Gln Val Gly Ser Gly Glu Gly Leu
           515                 520                 525
   Gly Val Ser Pro Ser Lys Ala Cys Gln Pro Ser Lys
       530                 535                 540
<210> 4
   <211> 1269
   <212> DNA
   <213> Sphingopyxis sp.
<220>
   <221> Seq ID 18 (fumI)
   <222> (1)..(1269)
<300>
   <308> FJ426269
   <309> 2009-06-11
<313> (1)..(1269)
<400> 4
   atggcgaacg gaacaaggca gaaagatctc agagaacgcg ccgaacgggt cattccgggc      60
   gggatgtacg gccacgagtc gacacggttg ctgccgccag aattccccca gttcttcagg     120
   cgcgcgctgg gggcacgaat ttgggacgcc gacgagcagc cctatatcga ctatatgtgc     180
   gcgtatgggc caaatttgct cggttaccgg caatccgaaa tcgaagccgc ggctgatgcg     240
   cagcgacttc tcggcgacac catgaccggt ccttcggaga tcatggtcaa cctcgccgaa     300
   gcctttgtgg gcatggtccg tcatgcggat tgggcgatgt tctgcaaaaa tggcagcgat     360
   gccacctcaa cggcgatggt tctcgcgcgt gcccatacgg ggcgcaaaac catattatgc     420
   gccaaaggcg cctatcatgg cgcttccccg tggaacactc cgcatactgc cgggattctc     480
   gcttccgatc gcgtgcatgt cgcatattat acctataacg acgcccaaag cttatcggac     540
   gcgttcaagg cgcacgatgg cgatattgcg gctgtctttg ccacaccttt ccgacacgaa     600
   gtatttgagg accaggccct cgcccagctt gagttcgcgc gcaccgctcg aaaatgttgt     660
   gacgagaccg gtgcgcttct ggtcgttgac gatgtgcgcg caggtttccg ggtggcgcgc     720
   gattgcagct ggacgcattt gggtatcgaa cccgatctca gttgctgggg aaaatgcttt     780
   gcgaatggct atccgatctc cgccctgctg ggctcgaaca aggcgcgcga tgcggcgcgg     840
   gatatatttg tgaccggctc cttctggttc tctgcggtac cgatggcggc cgcgatcgaa     900
   accctcagga tcattcgaga gacgccttat ctcgaaacgc tgatcgccag cggcgccgcc     960
   ctgcgggcag gcctggaggc acagtctcag cgccatggtc ttgagttgaa gcagacgggc    1020
   ccggcgcaga tgccgcaaat attctttgcg gacgatcccg attttcggat cggctatgcg    1080
   tgggccgcgg cgtgcctgaa gggcggcgtc tatgttcatc cctatcacaa tatgtttctc    1140
   tctgcggccc atacagttga cgatgtaacg gagaccctcg aggcgacgga tcgcgcgttc    1200
   agcgcggtcc tcagagattt tgcgtctctc cagcctcatc ccattttaat gcaactcgcc    1260
   ggtgcttga                                                            1269
<210> 5
   <211> 422
   <212> PRT
   <213> Sphingopyxis sp.
<220>
   <221> Seq ID 19 (FumI)
   <222> (1)..(422)
<300>
   <308> FJ426269
   <309> 2009-06-11
<313> (1)..(422)
<400> 5
   Met Ala Asn Gly Thr Arg Gln Lys Asp Leu Arg Glu Arg Ala Glu Arg
   1               5                   10                  15
   Val Ile Pro Gly Gly Met Tyr Gly His Glu Ser Thr Arg Leu Leu Pro
               20                  25                  30
   Pro Glu Phe Pro Gln Phe Phe Arg Arg Ala Leu Gly Ala Arg Ile Trp
           35                  40                  45
   Asp Ala Asp Glu Gln Pro Tyr Ile Asp Tyr Met Cys Ala Tyr Gly Pro
       50                  55                  60
   Asn Leu Leu Gly Tyr Arg Gln Ser Glu Ile Glu Ala Ala Ala Asp Ala
   65                  70                  75                  80
   Gln Arg Leu Leu Gly Asp Thr Met Thr Gly Pro Ser Glu Ile Met Val
                   85                  90                  95
   Asn Leu Ala Glu Ala Phe Val Gly Met Val Arg His Ala Asp Trp Ala
               100                 105                 110
   Met Phe Cys Lys Asn Gly Ser Asp Ala Thr Ser Thr Ala Met Val Leu
           115                 120                 125
   Ala Arg Ala His Thr Gly Arg Lys Thr Ile Leu Cys Ala Lys Gly Ala
       130                 135                 140
   Tyr His Gly Ala Ser Pro Trp Asn Thr Pro His Thr Ala Gly Ile Leu
   145                 150                 155                 160
   Ala Ser Asp Arg Val His Val Ala Tyr Tyr Thr Tyr Asn Asp Ala Gln
                   165                 170                 175
   Ser Leu Ser Asp Ala Phe Lys Ala His Asp Gly Asp Ile Ala Ala Val
               180                 185                 190
   Phe Ala Thr Pro Phe Arg His Glu Val Phe Glu Asp Gln Ala Leu Ala
           195                 200                 205
   Gln Leu Glu Phe Ala Arg Thr Ala Arg Lys Cys Cys Asp Glu Thr Gly
       210                 215                 220
   Ala Leu Leu Val Val Asp Asp Val Arg Ala Gly Phe Arg Val Ala Arg
   225                 230                 235                 240
   Asp Cys Ser Trp Thr His Leu Gly Ile Glu Pro Asp Leu Ser Cys Trp
                   245                 250                 255
   Gly Lys Cys Phe Ala Asn Gly Tyr Pro Ile Ser Ala Leu Leu Gly Ser
               260                 265                 270
   Asn Lys Ala Arg Asp Ala Ala Arg Asp Ile Phe Val Thr Gly Ser Phe
           275                 280                 285
   Trp Phe Ser Ala Val Pro Met Ala Ala Ala Ile Glu Thr Leu Arg Ile
       290                 295                 300
   Ile Arg Glu Thr Pro Tyr Leu Glu Thr Leu Ile Ala Ser Gly Ala Ala
   305                 310                 315                 320
   Leu Arg Ala Gly Leu Glu Ala Gln Ser Gln Arg His Gly Leu Glu Leu
                   325                 330                 335
   Lys Gln Thr Gly Pro Ala Gln Met Pro Gln Ile Phe Phe Ala Asp Asp
               340                 345                 350
   Pro Asp Phe Arg Ile Gly Tyr Ala Trp Ala Ala Ala Cys Leu Lys Gly
           355                 360                 365
   Gly Val Tyr Val His Pro Tyr His Asn Met Phe Leu Ser Ala Ala His
   370 375 380
   Thr Val Asp Asp Val Thr Glu Thr Leu Glu Ala Thr Asp Arg Ala Phe
   385 390 395 400
   Ser Ala Val Leu Arg Asp Phe Ala Ser Leu Gln Pro His Pro Ile Leu
   405 410 415
   Met Gln Leu Ala Gly Ala
   420

## Beschreibung

Die vorliegende Erfindung bezieht sich auf einen Zusatzstoff zum enzymatischen Abbau von Fumonisinen, in einem pflanzlichen Rohstoff und Mischungen, die pflanzliche Rohstoffe enthalten, sowie eine Verwendung desselben.

Mykotoxine treten auf landwirtschaftlichen, pflanzlichen Produkten sehr häufig auf und verursachen je nach Art der Mykotoxine schwere wirtschaftliche Schäden, insbesondere in den aus den landwirtschaftlichen Produkten hergestellten Nahrungsmitteln und auch bei mit derartigen Nahrungsmitteln ernährten Tieren und Menschen, wobei derartige Schäden äußerst vielfältig sind. Es wurden bereits zahlreiche Methoden entwickelt, mit welchen versucht wird, derartige Mykotoxine zu entgiften bzw. abzubauen oder unschädlich zu machen, um durch die Mykotoxine verursachte Schäden in den Bereichen von tierischer und menschlicher Ernährung, der Tierzucht, Verarbeitung von Futter- und Lebensmitteln und dgl. hintanzuhalten.

Unter den bekannten Mykotoxinen existiert eine Vielzahl von untereinander strukturell verwandten Mykotoxinen, wie beispielsweise die Fumonisine, von welchen Fumonisin B1 das am häufigsten vorkommende Toxin der Gruppe ist. Jedoch sind zahlreiche Derivate und verwandte Moleküle bekannt, welche ebenfalls giftige Wirkungen bei Menschen und Tieren aufweisen. So ist es bekannt, dass die Fumonisine den Sphingolipidstoffwechsel durch eine Wechselwirkung mit dem Enzym Ceramidsynthase behindern. Sphingolipide sind nicht nur Bestandteil von Zellmembranen, sondern spielen auch eine wichtige Rolle als Signal- und Botenmoleküle in vielen elementaren, zellulären Prozessen, wie dem Zellwachstum, der Zellwanderung und Zellanbindung, bei entzündlichen Prozessen und intrazellulären Transportvorgängen. Aufgrund dieser Behinderung des Sphingolipidstoffwechsels werden Fumonisine für die giftige Wirkung auf unterschiedlichste Tierarten und auch für den Menschen verantwortlich gemacht. So konnte nachgewiesen werden, dass Fumonisine bei Nagetieren krebserregend wirken, und sie wurden durch epidemiologische Daten mit Speiseröhrenkrebs und dem Neuralrohrdefekt bei Menschen in Zusammenhang gebracht. Bei verschiedenen Tierarten, wie beispielsweise Schweinen, werden sie für die typische Toxikose durch Lungenödeme verantwortlich gemacht. Fumonisine sind in diesem Zusammenhang eine nahezu allgegenwärtige Kontamination auf verschiedensten Getreidearten und insbesondere auf Mais sowie auf Nüssen und Gemüse sind, ist dieser stark negative Effekt in Bezug auf die Gesundheit von Menschen und Tieren nicht vernachlässigbar.

Der mikrobielle Abbau von Fumonisinen wurde bereits in der EP-A 1 860 954 beschrieben, gemäß welcher Mikroorganismen zur Entgiftung von Fumonisinen und Fumonisinderivaten eingesetzt werden, bei welchen die detoxifizierenden Bakterien oder Hefen, gewählt aus genau definierten Stämmen zur Entgiftung von Fumonisinen, Futtermitteln zugesetzt werden.

Auch wurden bereits katabolische Stoffwechselwege für den biologischen Abbau von Fumonisinen und die hierfür verantwortlichen Gene und Enzyme beschrieben. So beschreibt beispielsweise die EP 0 988 383 Fumonisin entgiftende Zusammensetzungen und Verfahren, wobei die eingesetzten, Fumonisin abbauenden Enzyme in erster Linie in transgenen Pflanzen produziert werden, bei welchen die Entgiftung von Fumonisinen mit Hilfe einer Aminooxidase, welche für ihre enzymatische Aktivität molekularen Sauerstoff benötigt, erfolgt.

Des Weiteren beschreibt die WO 2004/085624 Transaminasen, Deaminasen und Aminomutasen und Zusammensetzungen und Verfahren zur enzymatischen Detoxifizierung, wobei insbesondere aminierte Toxine, beispielsweise Fumonisine, entgiftet werden. In diesem Zusammenhang werden Polypeptide, welche eine Deaminaseaktivität besitzen, zur Entgiftung eingesetzt.

Aus der WO 00/04158 ist die Verwendung von Fumonisin abbauenden Aminooxidasen bei der Herstellung von Nahrungs- oder Futtermitteln bzw. bei der Verarbeitung von pflanzlichen Rohstoffen bekannt geworden.

Bisher bekannten Verfahren ist jedoch gemeinsam, dass sie für eine Detoxifizierung der Mykotoxine molekularen Sauerstoff für die beschriebenen, katabolischen Stoffwechselwege benötigen, wobei die insbesondere erforderlichen Aminooxidasen unter sauerstoffunabhängigen Bedingungen nicht arbeiten können. Ein Einsatz von derartigen Genen und Enzymen zur Detoxifizierung von Futtermitteln, beispielsweise im Verdauungstrakt von Tieren, ist aufgrund des im wesentlichen sauerstofffreien Milieus in dem Verdauungstrakt von Tieren nicht möglich bzw. zeigen die bekannten Gene und Enzyme keinerlei Wirkung.

Die Erfindung zielt nun darauf ab, einen Zusatzstoffes für den enzymatischen Abbau von Mykotoxinen zur Verfügung zu stellen, mit welchem es sicher und zuverlässig gelingt, Fumonisine zu toxikologisch unbedenklichen Substanzen abzubauen bzw. zu entgiften.

Als pflanzliche Rohstoffe werden hierbei Getreide bzw. Getreideprodukte, Gräser, Obst oder Gemüse sowie Zwischenprodukte die diese Stoffe zur Herstellung von Nahrungs- und Futtermittel enthalten, wie beispielsweise Silage, Obstmaische oder dgl. verstanden.

Als Zusatzstoffe werden vor allem Futtermittelzusätze, Nahrungsmittelzusätze sowie Zusätze zur Bioethanolherstellung verstanden.

Mit einem derartigen Verfahren gelingt es weiterhin, den Sphingolipidstoffwechsel, der durch eine Wechselwirkung der Fumonisine mit dem Enzym Ceramidsynthase behindert wird, aufrecht zu erhalten und gleichzeitig die Fumonisine biologisch zu nicht toxischen Substanzen abzubauen. Schließlich können technologische Anwendungen der Detoxifizierung erzielt werden, da dieses Verfahren auch im größeren technischen Maßstab anwendbar ist, so daß mit dem erfindungsgemäßen Verfahren sicher und zuverlässig mykotoxinfreie Produkte hergestellt werden können.

Die in dem Zusatzstoff eingesetzten Nukleinsäuresequenzen bzw. die mittels dieser Nukleinsäuresequenzen in prokaryotischen und eukaryotischen Wirtszellen exprimierten, in sauerstoffunabhängigem Milieu katalytisch wirkenden Enzyme sind nachfolgend aufgelistet.

### Nukleinsäuren

### Sequenzen:

SEQ ID No 1
   >Seq ID 1 (fum (Fumonisinkatabolismus) Gencluster, 15.420 bp)
SEQ ID No 2
   >Seq ID 8 (*fumD*)
SEQ ID No 4
   >Seq ID 18 (*fumI*)

### Sequenzen:

SEQ ID No 3
   >Seq ID 9 (FumD)
SEQ ID No 5
   >Seq ID 19 (FumI)

Zur Lösung dieser Aufgaben ist ein derartiger Zusatzstoff dadurch gekennzeichnet, dass ein Enzym enthalten ist, welches wenigstens 90 % Sequenzidentität mit einem Enzym der SEQ ID No 3 (Seq ID 9 (FumD)) aufweist, sowie gegebenenfalls zusätzlich ein Cosubstrat für das eingesetzte Enzym, ein Enzym der SEQ ID No 5 (Seq ID 19 (FumI)) und ein inerter Träger enthalten sind.

Mit einem derartigen Zusatzstoff gelingt es, einerseits beispielsweise Mykotoxine direkt auf dem Rohmaterial vollständig und zuverlässig abzubauen, wobei die spezifischen bei diesem Verfahren produzierten Enzyme den Abbau von Fumonisinen und von Zwischenprodukten des Abbauweges katalysieren, und andererseits Mykotoxine beispielsweise auch direkt bei der Bioethanolherstellung in der Maische für die Alkoholherstellung abzubauen oder auch bei der Herstellung von Nahrungsmitteln direkt in dem Herstellverfahren abzubauen bzw. unschädlich zu machen.

Ein derartiger Zusatzstoff, in dem ein Enzym eingesetzt ist, welches wenigstens 90 % Sequenzidentität mit dem Enzym, der SEQ ID No 3 (Seq ID 9 (FumD)) oder ein kompletter, rekombinanter Wirtsorganismus zur Expression dieses Enzyms, sowie gegebenenfalls zusätzlich wenigstens ein Cosubstrat für das eingesetzte Enzym, sowie ein Enzym der SEQ ID No 5 (Seq ID 19 (FumI) und ein inerter Träger enthalten sind, zeichnet sich dadurch aus, dass er zielgerichtet Fumonisine abbaut und somit detoxifiziert. Durch den Einsatz eines erfindungsgemäßen Zusatzstoffes, welcher im Wesentlichen aus einem isolierten Enzym sowie gegebenenfalls dessen Cosubstrat und Trägern besteht, ergibt sich der Vorteil, dass dieses seine katalytische Aktivität in einer Umgebung und unter Bedingungen beibehält, in welchem beispielsweise komplette Mikroorganismen nicht oder nur wenig aktiv wären, wobei gleichzeitig eine bedeutend höhere, spezifische Aktivität erzielt werden kann, sowie definierte Reaktionen unter Vermeidung von unerwünschten Nebenreaktionen katalysiert werden können.

Darüber hinaus können Probleme, welche gemäß dem Stand der Technik durch den Einsatz vermehrungsfähiger Keime auf landwirtschaftlichen Rohprodukten entstanden sind, mit Sicherheit hintangehalten werden und überdies weisen Zusatzstoffe, welche nur isolierte Enzyme enthalten, sowohl eine bessere Eignung zur Formulierung für eine gezielte und kontrollierte Aktivierung, d.h. beispielsweise an einer bestimmten Stelle des Verdauungstrakts, als auch die Vermeidung von unerwünschtem, erhöhtem Substratverbrauch auf. Um die Spezifität noch weiter zu erhöhen, ist der Zusatzstoff dahingehend weitergebildet, dass ein durch molekulargenetische Methoden, Mutagenese oder molekulare Evolution verändertes Enzym eingesetzt ist.

Wenn ein Enzym eingesetzt ist bzw. wird, welches wenigstens 90 % Sequenzidentität mit einem Enzym mit der SEQ ID No 3 (Seq ID 9 (FumD) aufweist, gelingt es, neben den bevorzugt abzubauenden Fumonisinen auch weitere Mykotoxine sicher und zuverlässig sauerstoffunabhängig abzubauen, wodurch eine weitgehende Entgiftung der beispielsweise auf pflanzlichen Rohprodukten vorhandenen Fumonisine, erzielt werden kann.

Indem der Zusatzstoff so ausgebildet ist, wie dies einer bevorzugten Weiterbildung der Erfindung entspricht, dass das Enzym und/oder die zu wenigstens 90 % identen Enzyme mit einer Schutzhülle ummantelt eingesetzt sind, kann sichergestellt werden, dass das Enzym oder die zu wenigstens 90 % identischen Enzyme vor einem vorzeitigen Aktivitätsverlust gesichert sind und sicher und zuverlässig an der vorgesehenen Stelle beispielsweise im Magen-Darm-Trakt ihre Wirkung entfalten.

Indem der Zusatzstoff bevorzugt so weitergebildet ist, dass das Enzym aus einer Carboxylesterase SEQ ID No 3 (Seq ID 9 (FumD)) gewählt ist, wird im Wesentlichen ein zum Substratkatabolismus befähigtes Enzym zum Einsatz gebracht, so dass neben einer geringeren Menge an einzusetzendem Enzym auch sichergestellt werden kann, dass keine unerwünschten Nebenreaktionen bei Einsatz des Enzyms auftreten.

Gemäß einer Weiterbildung der Erfindung wird eine Verwendung des Zusatzstoffes in einem sauerstoffunabhängigen Milieu bei der Bioethanolherstellung, gemeinsam mit einer Maische bzw. einem pflanzlichen Ausgangsmaterial, durchgeführt, wobei der Zusatzstoff so gewählt ist, dass das darin enthaltene Enzym vollständig aus Bakterien stammt, das den Katabolismus von Fumonisinen über einen hochspezifischen Abbauweg katalysiert, wodurch es gelingt, es mit hoher Spezifität, Aktivität und Effizient einzusetzen, wodurch der Zusatzstoff auch in einem sauerstoffunabhängigen Milieu technologisch verwendet werden kann.

Gemäß einer bevorzugten Verwendung wird der Zusatzstoff gemäß der Erfindung zur sauerstoffunabhängigen bzw. anaeroben Behandlung eines pflanzlichen Ausgangsmaterials bzw. einer Maische in der Bioethanolherstellung eingesetzt. In diesem Fall gelingt es, die in dem pflanzlichen Ausgangsmaterial bzw. Rohstoff enthaltenen Mykotoxine während der Bioethanolherstellung im sauerstoffunabhängigen Milieu sicher und zuverlässig unschädlich zu machen, so dass der Rückstand aus der Ethanolproduktion, nämlich der Trester oder die Trockenschlempe, nachfolgend entweder direkt oder nach Trocknung und Pelletierung ohne weitere Verarbeitung, insbesondere Detoxifizierung, als Futtermittel, welches frei von Fumonisinen ist, eingesetzt werden kann.

Die Erfindung wird nachfolgend anhand von Ausführungsbeispielen sowie Figuren näher dargestellt. In diesen zeigt:
Fig. 1 den Fumonisin-katabolischen Gencluster,
Fig. 2 die Michaelis-Menten-Kurve für Fumonisin-Carboxylesterase FumD,
Fig. 3 eine Abbaukurve von hydrolysiertem Fumonisin B₁,
Fig. 4 die Umwandlung von Fumonisin FB1 in hydrolysiertes Fumonisin HFB 1 nach Zugabe von Carboxylesterase SEQ ID No 3 (Seq ID 9 (FumD))zeigt, und
Fig. 5 den Abbau von hydrolysiertem Fumonisin HFB1 durch Zusatz von Aminotransferase SEQ ID No 5 (Seq ID 19 (FumI)).

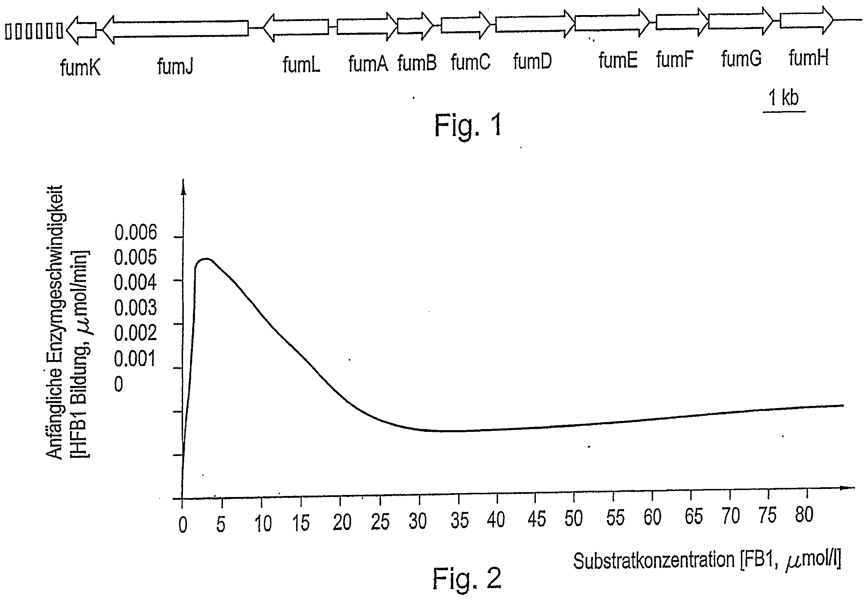

In Fig. 1 ist ein Fumonisin katabolischer Gencluster als Teilsequenz von 15420 Basenpaaren eines mikrobiellen Stammes mit der Hinterlegungsnummer DSM 16254 dargestellt. In dem fum-Gencluster des prokaryotischen Stammes DSM 16254 wird die Transkription der offenen Leserahmen durch einen bidirektionalen Promotor gesteuert bzw. geregelt, welcher zwischen *fumA* und *fumI* angeordnet ist. Der Cluster codiert Proteine, welche in der Regulierung der Genexpression, wie beispielsweise FumB und FumC, in der Substraterkennung und dem Transport, wie beispielsweise FumJ, FumA und FumG und in einem Substratkatabolismus, wie beispielsweise FumD, FumE, FumF, FumH, FumI und FumK involviert sind.

### Beispiele

### Beispiel 1: Die Enzymkinetik von Fumonisincarboxylesterase.

Das *fumD* Gen (SEQ ID No 2), welches eine Fumonisincarboxylesterase codiert, wurde kloniert und in *Pichia pastoris* unter Verwendung von Standardverfahren exprimiert. Das hisgetaggte Enzym wurde rückgewonnen und aus der überstehenden Lösung der Kultur durch Affinitätschromatographie gereinigt. Die Enzymkonzentration wurde bestimmt und die enzymkinetischen Parameter wurden mit sieben unterschiedlichen Substratkonzentrationen im Bereich zwischen 50 µg bis 25 mg FB₁ pro Liter und einer Enzymkonzentration von 0,33 ng/ml bestimmt. Die Reaktionen wurden in 20 mM Tris-Cl (pH 8,0) Puffer mit 0,1 mg/ml Rinderserumalbumin gepuffert und bei 30 °C inkubiert. Proben wurden nach 0, 30, 60 120 und 240 Minuten Inkubation genommen und durch HPLC-MS/MS analysiert. Fumonisin B₁ (FB₁) und hydrolysiertes Fumonisin B₁ wurden quantifiziert basierend auf einer Kalibrierung mit den gereinigten Referenzsubstanzen und einem vollständig ¹³C markierten, internen FB1 Standard.

Fig. 2 zeigt die Michaelis-Menten-Kurve für die Hydrolyse von Fumonisin B1 (FB1) durch Fumonisin-Carboxylesterase FumD, welcher bei einer Enzymkonzentration von 0,33 ng/ml in Tris-Cl-Puffer (pH 8,0) bestimmt wurde, wobei anfängliche Enzymgeschwindigkeiten gegen die Substratkonzentration aufgetragen wurden. Die Michaelis-Menten-Kurve zeigt einen Abfall bei höheren Substratkonzentrationen, da die Enzymgeschwindigkeit basierend auf dem Produkt, d.h. hydrolysierter FB₁-Bildung berechnet wurde. Da hydrolysiertes FB₁ aus FB₁ in einer zweistufigen Reaktion über partiell hydrolysiertes FB₁ mit lediglich einer Tricarballylsäure-Seitenkette, die zurückbehalten wurde, und einer Seitenkette ausgebildet wird, die abgespalten wurde, war die Endproduktbildung bei hohen Substratkonzentrationen verzögert. Die Michaelis-Menten-Konstante K_{M} wurde als 0,90 µmol/l berechnet, was 650 ppb äquivalent ist, und die Umwandlungszahl war 900 pro Sekunde.

Aus Fig. 2 ergibt sich, dass Fumonisine mit der Carboxylesterase in den relevanten Konzentrationsbereichen rasch und vollständig hydrolysiert werden können.

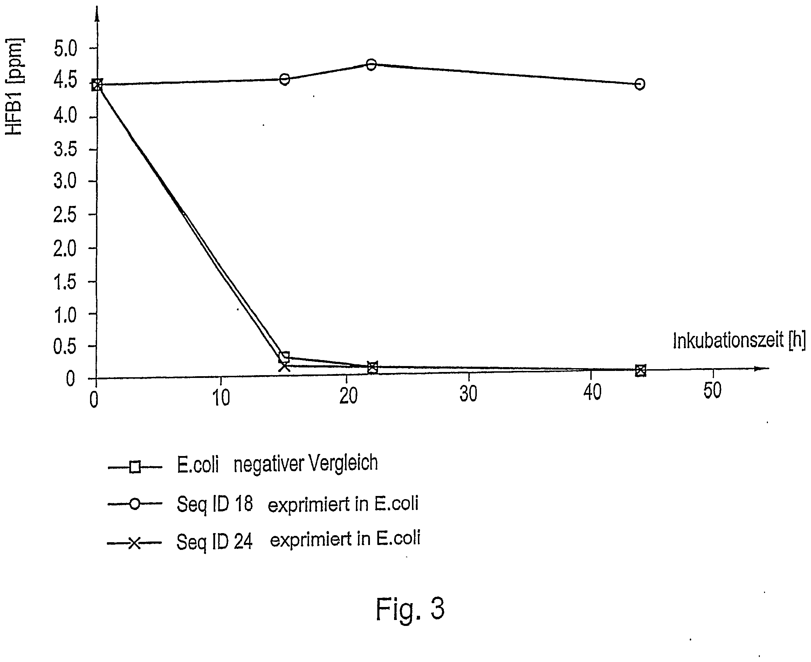

Beispiel 2: Die katalytische Aktivität von HFB1 (hydrolysiertes Fumonisin B1) Aminotransferase SEQ ID No 4 wurde unter Verwendung von Standardverfahren kloniert und in *E. coli* exprimiert unter Steuerung bzw. Regelung eines Bakteriophagen T7 Promotors. Die Bakterienzellen wurden gesammelt in 50 mM Natriumphosphatpuffer, neuerlich suspendiert und durch Ultraschall lysiert. Hydrolysiertes Fumonisin wurde hinzugefügt und die Proben wurden bei 25 °C inkubiert. Proben wurden in Zeitintervallen genommen und durch HPLC-MS/MS analysiert. Es wurde keine Reduktion der hydrolysierten FB₁ Konzentration beobachtet. Wenn ein Cosubstrat, wie beispielsweise eine α-Ketosäure, wie Brenztraubensäure oder Oxalacetat, zu der Reaktion hinzugefügt wurde, konnte ein vollständiger Abbau des hydrolysierten Fumonisins zu 2-Keto-HFB₁ beobachtet werden, wie dies in Fig. 3 dargestellt ist. Diese Substanz ist für Säuger vollständig unschädlich.

### Beispiel 3: Enzymaktivität in Darmmilieu

Zur Überprüfung der enzymatischen Aktivität von FUM-Carboxylesterase im Verdauungstrakt wurden schlachtfrische Schweinedärme verwendet und unter Ausschluss von Sauerstoff ins Labor transportiert und in einer anaeroben Sterilwerkbank untersucht. Etwa 10 cm lange Stücke von Duodenum und Jejunum wurden abgebunden und herausgeschnitten. Mit Kanülen wurde Fumonisin B1 in konzentrierter wässriger Lösung auf eine Endkonzentration von etwa 10 ppm verdünnt eingespritzt und mit Darminhalt vermischt. Anschließend wurden 5 µg Fumonisin-Carboxylesterase in wässriger Lösung, bzw. dasselbe Volumen Wasser in den Negativkontrollen, eingespritzt und eingemischt. Die Darmabschnitte wurden bei 39 °C inkubiert. Mit Hilfe von Kanülen wurden Proben gezogen und durch HPLC-MS/MS analysiert. Dabei zeigte sich, dass Fumonisin B1 im Duodenum und Jejunum zum Zeitpunkt der ersten Probenahme nach zwei Stunden bereits vollständig hydrolysiert war.

### Beispiel 4: Ermittlung des Temperaturbereichs der Aktivität von Fumonisin-Carboxylesterase

Zur Ermittlung des Temperaturbereichs, in dem Fumonisin-Carboxylesterase aktiv ist, wurden 1,6 ng/ml FUM-Carboxylesterase in 20 mM Tris-Cl Puffer, pH 7,0, mit 0,1 mg/ml BSA und 10 ppm Fumonisin B1 bei unterschiedlichen Temperaturen inkubiert. Dabei zeigte sich, dass das Temperaturoptimum für das Enzym bei 30 °C lag. Auch bei 40 °C und sogar 50 °C wurde enzymatische Aktivität noch eindeutig festgestellt. Somit ist diese FUM-Carboxylesterase zur Anwendung unter den Temperaturbedingungen wie im Verdauungstrakt, oder im Zuge von Prozessschritten der Lebens- oder Futtermittelherstellung, die bei erhöhter Temperatur stattfinden, geeignet.

### Beispiel 5: Bestimmung des pH-Bereichs der Aktivität von Fumonisin-Carboxylesterase

Zur Bestimmung des pH-Bereichs, in dem Fumonisin-Carboxylesterase aktiv ist, wurde Teorell-Stenhagen-Puffer verwendet. Dieser Puffer lässt sich durch die Kombination von Citrat, Phosphat und Borat über einen Bereich von 10 pH Einheiten mit gleicher Pufferkapazität einstellen. FUM-Carboxylesterase wurde in einer Konzentration von 3,3 ng/ml mit 10 ppm Fumonisin B1 bei verschiedenen pH-Werten in diesem Puffer bei 25 °C inkubiert. Die höchste Aktivität zeigte sich bei pH 8,0, aber es konnte im gesamten Bereich von pH 5 bis pH 10 Aktivität festgestellt werden. Durch die Aktivität in diesem breiten pH-Bereich wird die technologische Anwendung des Enzyms als Futtermittelzusatz bzw. im Zuge der Verarbeitung von Lebens-und Futtermitteln ermöglicht.

### Beispiel 6: Fütterungsversuch mit Ferkeln

Der Versuch wurde in einem Versuchsstall mit 12 Buchten für jeweils 10 Tiere durchgeführt. Der Stall war ausgestattet mit Spaltenboden, Schalentränkern und einem computergesteuerten Fütterungssystem. Die Automaten waren entlang der Buchtenwände angeordnet. Das Stallklima wurde täglich automatisch aufgezeichnet und die Temperatur nach den Standardempfehlungen zur Ferkelaufzucht eingestellt.

120 gemischt-geschlechtliche Absetzferkel (Alter: ca. 4 Wochen, durchschnittliches Einstellgewicht 8,21 kg) wurden für diesen Versuch eingesetzt. Jedes Ferkel wurde mit einer Ohrmarke versehen und einzeln gewogen. Die 120 Ferkel wurden auf 12 Buchten zufällig verteilt. Alle Ferkel entstammten dem österreichischen Zuchtprogramm ÖHYB (= (Edelschwein x Landrasse) x Pietrain).

Direkt nach dem Absetzen wurden die Ferkel für 2 Tage mit einem Starterfutter gefüttert, nach dieser Eingewöhnungsphase erfolgte die Umstellung auf das Versuchsfutter. Die Fütterung erfolgte 2-phasig: Absetzphase Tag 1 - 14, Aufzuchtphase Tag 15 - 42. Das Versuchsfutter wurde buchtenindividuell über die Spotmix-Fütterungsanlage gemischt und je nach Anzahl der Ferkel, Gewichtsentwicklung und Futterverzehr zweimal täglich trocken zugeteilt. Wasser stand zur Aufnahme ad libitum zur Verfügung. Die 12 Buchten wurden in vier verschiedene Applikationsgruppen zu je drei Wiederholungen geteilt und erhielten die folgenden Einmischungen ins oben beschriebene Futter:

| Gruppe | |
|---|---|
| Negativ-Kontrolle | Keine Toxine, kein Enzymzusatz |
| Positiv-Kontrolle | 4 - 5,5 ppm Fumonisin B1 |
| Versuchsgruppe 1 | 4 - 5,5 ppm Fumonisin B1 + Enzymmix 1 (Carboxylesterase, Aminotransferase, Pyruvat) 0,5 kg/t Futter |
| Versuchsgruppe 2 | 4 - 5,5 ppm Fumonisin B1 + Enzymmix 1 (Carboxylesterase, Aminotransferase, Pyruvat, inerter Carrier) 1 kg/t Futter |

In der Positiv-Kontrolle wurden bei fast der Hälfte der Tiere respiratorische Probleme beobachtet, wobei es auch zu einem Ausfall kam. Alle anderen Gruppen erschienen gesund.

**Leistungsdaten**

| Gruppe | Anzahl der Tiere | Anfangsgewicht (Durchschnitt, kg) | Endgewicht (Durchschnitt, kg) | Ausfälle |
|---|---|---|---|---|
| Negativ-Kontrolle | 30 | 8,34 | 26,82 | |
| Positiv-Kontrolle | 30 | 8,17 | 24,77 | 1 |
| Versuchsgruppe 1 | 30 | 8,08 | 26,69 | |
| Versuchsgruppe 2 | 30 | 8,25 | 27,03 | |

### Beispiel 7: Enzymatischer Abbau von Fumonisinen in der Bioethanolmaische

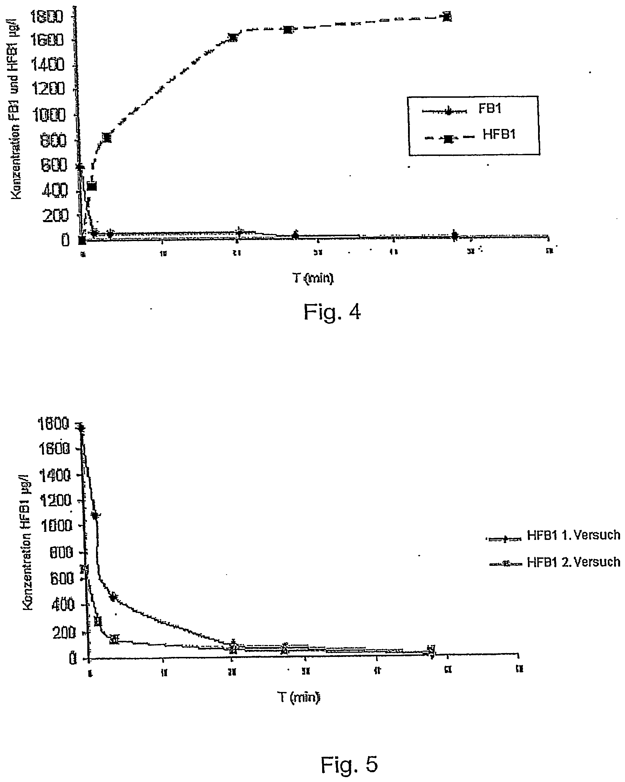

Proben von Maismaische zur Bioethanolherstellung wurden genommen und bei 30 bis 65 °C unter Rühren inkubiert, wobei der Abbau von Fumonisin B1 nach Zugabe von 770 Units Carboxylesterase SEQ ID No 3 (Seq ID 9 (FumD)) pro Kubikmeter Maische unter Rühren (Rührzeit in Minuten) untersucht wurde. Proben wurden nach der Probennahme durch Aufkochen inaktiviert und danach für die Analytik abzentrifugiert und ein Aliquot des Überstandes abgedampft. Der Rückstand wurde in 200 µl Probenpuffer, der C13-markierten, internen Fumonisin-Standard enthielt, aufgenommen, 1,5 min geschüttelt, abzentrifugiert und dann einer LC-MS-Analyse unterzogen. Hieraus ergibt sich, dass, wie dies in Fig. 4 dargestellt ist, Fumonisin FB1 vollständig in hydrolysiertes Fumonisin HFB1 umgewandelt wird. Nach Zusatz von Aminotransferase SEQ ID No 5 wird das hydrolysierte Fumonisin HFB1 vollständig zu unschädlichen Bestandteilen abgebaut, wie dies in Fig. 5 dargestellt ist.

### Beispiel 8: Abbau von Fumonisinen und ihren Derivaten in Maistortillabrei und Cornflakesbrei

Die Wirksamkeit der Fumonisin-abbauenden Enzyme wurde in Maisbreiproben ("corn grits") für die Maistortilla- und Cornflakes-Herstellung untersucht. Fumonisin-kontaminierter Mais (ca. 1 ppm) wurde zu Maismehl vermahlen, mit Wasser versetzt und aufgekocht. Für die Tortilla-Herstellung wurde der auf ca. 50 bis 60 °C abgekühlte Maisbrei mit einer Mischung von Proteinasen in alkalischer Lösung versetzt. Nach 30 bis 180 min, wenn der pH unter 9, vorzugsweise unter pH 8, gefallen ist, wurde eine Mischung aus Carboxylesterase und Aminotransferase (jeweils 500 - 1000 Units/m³) hinzugefügt und für weitere 30 bis 60 min inkubiert. Im Falle der Cornflakes-Herstellung wurde ein Maisbrei aus gemahlenem Mais und Gerstenmalz für ca. eine Stunde im Druckgefäß aufgekocht; nach Abkühlung unter 60 °C (vorzugsweise 50 °C) wurde eine Enzymmischung, bestehend aus Carboxylesterase und Aminotransferase (jeweils 500 - 1000 Units/m³), hinzugefügt und für weitere 30 bis 60 min inkubiert. Aus dieser Mischung wurden dann Proben gezogen und auf FB1- bzw. HFB1-Rückstände wie in Beispiel 7 untersucht. HFB1-Levels waren in sämtlichen Proben unter 80 ppb, offensichtlich wurde das aus FB1 entstandene HFB1 kontinuierlich weiter umgesetzt. Die gemessenen Werte für FB1 sind in der u.a. Tabelle angeführt.

**Tabelle: Enzymatischer Abbau von FB1 und HFB1 in Maisbrei; Fumonisinkonzentration in ppb (µg/kg)**

| Behandlungszeit mit Enzymmix (min) | Tortillabrei (40 °C, 500 Units) | Tortillabrei (50 °C, 1000 Units) | Cornflakesbrei (35 °C, 500 Units) | Cornflakesbrei (40 °C, 1000 Units) |
|---|---|---|---|---|
| 0 | 852 | 866 | 912 | 1053 |
| 10 | 116 | 134 | 51 | 97 |
| 30 | 32 | 71 | 17 | 37 |

## Patentansprüche

1. Zusatzstoff geeignet zum enzymatischen Abbau von Fumonisinen, in einem pflanzlichen Rohstoff und Mischungen, die pflanzliche Rohstoffe enthalten, **dadurch gekennzeichnet, dass** ein Enzym enthalten ist, welches wenigstens 90 % Sequenzidentität mit einem Enzym der SEQ ID No 3 (Seq ID 9 (FumD)) aufweist, sowie gegebenenfalls zusätzlich ein Cosubstrat für das eingesetzte Enzym, ein Enzym der SEQ ID No 5 (Seq ID 19 (FumI)) und ein inerter Träger enthalten sind.

2. Zusatzstoff nach Anspruch, **dadurch gekennzeichnet, dass** das Enzym und/oder die zu wenigstens 90 % identischen Enzyme mit einer Schutzhülle ummantelt eingesetzt sind.

3. Zusatzstoff nach Anspruch 1 oder 2, **dadurch gekennzeichnet, dass** das Enzym aus einer Carboxylesterase SEQ ID No 3 (Seq ID 9 (FumD)), gewählt ist.

4. Verwendung eines Zusatzstoffes nach einem der Ansprüche 1 bis 3, **dadurch gekennzeichnet, dass** der Zusatzstoff in einem sauerstoffunabhängigen Milieu bei der Bioethanolherstellung, gemeinsam mit einer Maische bzw. einem pflanzlichen Ausgangsmaterial, eingesetzt ist.

5. Verwendung nach Anspruch 4 zur sauerstoffunabhängigen Behandlung eines pflanzlichen Ausgangsmaterials bzw. einer Maische in der Bioethanolherstellung.

## Claims

1. An additive suitable for the enzymatic degradation of fumonisins in a vegetable raw material and mixtures containing vegetable raw materials, **characterized in that** an enzyme comprising at least 90% sequence identity with an enzyme of SEQ ID NO:3 (SEQ ID NO:9 (FumD)) is contained, and optionally a cosubstrate for the used enzyme, an enzyme of SEQ ID NO:5 (SEQ ID:19 (FumI)) and an inert carrier are additionally contained.

2. An additive according to claim 1, **characterized in that** the enzyme and/or the at least 90% identical enzymes are used coated with a protective cover.

3. An additive according to claim 1 or 2, **characterized in that** the enzyme is selected from a carboxylesterase SEQ ID NO:3 (SEQ ID NO:9 (FumD)).

4. The use of an additive according to any one of claims 1 to 3, **characterized in that** said additive is used in an oxygen-independent environment in the production of bioethanol, along with a mash and/or a vegetable starting material.

5. The use according to claim 4 for the oxygen-independent treatment of a vegetable starting material or a mash in the production of bioethanol.

## Revendications

1. Additif approprié pour la décomposition enzymatique de fumonisines dans une matière première végétale et des mélanges qui contiennent des matières premières végétales, **caractérisé en ce qu'**est contenue une enzyme, laquelle présente une identité de séquence d'au moins 90 % avec une enzyme de SEQ ID No 3 (Seq ID 9 (FumD)), et que, le cas échéant, sont contenus également un co-substrat pour l'enzyme utilisée, une enzyme de SEQ ID No 5 (Seq ID 19 (FumI)) et un support inerte.

2. Additif selon la revendication, **caractérisé en ce que** l'enzyme et/ou l'enzyme identique à au moins 90% est utilisée revêtue d'une enveloppe protectrice.

3. Additif selon la revendication 1 ou 2, **caractérisé en ce que** l'enzyme est choisie à partir d'une carboxylestérase SEQ ID No 3 (Seq ID 9) (FumD)).

4. Utilisation d'un additif selon l'une des revendications 1 à 3, **caractérisée en ce que** l'additif est utilisé dans un milieu indépendant de l'oxygène lors de la production de bioéthanol, conjointement avec un moût ou un matériau de départ végétal.

5. Utilisation selon la revendication 4, pour le traitement indépendant de l'oxygène d'un matériau de départ végétal ou d'un moût dans la production de bioéthanol.
